# FASCICULE DE BREVET EUROPEEN

(11) **EP 2 983 792 B1**
(45) Date de publication et mention de la délivrance du brevet: **03.05.2017**
(21) Numéro de dépôt: 14719040.9
(22) Date de dépôt: 21.03.2014
(51) Int. Cl.: A61Q 5/00, A61K 8/81, A61Q 19/00, A61K 8/06, C08L 33/14, C08L 33/26, C08J 3/02

(54) **NOUVELLES ÉMULSIONS EAU-DANS-HUILE À FORTE TENEUR EN PHASE AQUEUSE, DE CONSISTANCES LIQUIDES ET STABLES AU STOCKAGE**
NEUARTIGE ÖL-IN-WASSER-EMULSIONEN MIT EINER HOCHWÄSSRIGEN PHASE, FLÜSSIGER KONSISTENZ UND LAGERSTABILITÄT
NOVEL OIL-IN-WATER EMULSIONS WITH A HIGH AQUEOUS PHASE CONTENT, LIQUID CONSISTENCIES AND STORAGE STABILITY

(30) Priorité: 12.04.2013 FR 1353352
(43) Date de publication de la demande: 17.02.2016
(73) Titulaire: Société d'Exploitation de Produits pour les Industries Chimiques SEPPIC, 75321 Paris Cedex 7 (FR)
(72) Inventeur: MERAT, Emmanuelle, F-81440 Lautrec (FR); SIGURANI, Séverine, F-81100 Castres (FR)
(74) Mandataire: Conan, Philippe Claude
(86) Numéro de dépôt international: PCT/FR2014/050668
(87) Numéro de publication internationale: WO 2014/167200

(56) Documents cités:
- EP-A1- 1 426 436
- FR-A1- 2 910 899

## Description

La présente invention a pour objet de nouvelles compositions se présentant sous la forme d'émulsions eau-dans-huile, ainsi que leurs utilisations en cosmétique, en pharmacie dans l'industrie textile, et dans l'industrie papetière.

Les compositions cosmétiques, dermocosmétiques, dermopharmaceutiques et pharmaceutiques peuvent se présenter sous forme de solutions aqueuses, d'émulsions, de poudres. Les émulsions sont particulièrement des formes privilégiées car elle permettent de véhiculer à la fois les substances hydrosolubles et des substances liposolubles qui sont couramment utilisées dans ces applications. On distingue les émulsions huile-dans-eau (H/E) dont la phase continue est constituée par une phase hydrophile, généralement une phase aqueuse, et la phase dispersée par une phase grasse lipophile, et les émulsions eau-dans-huile (E/H) dont la phase continue est constituée par une phase grasse lipophile et la phase dispersée par une phase hydrophile, généralement une phase aqueuse.

Les émulsions huile-dans-eau sont intrinsèquement plus stables que les émulsions eau-dans-huile ; les émulsions eau-dans-huile présentent néanmoins de nombreux avantages. En effet la séparation entre les gouttelettes d'eau réduit la possibilité de prolifération de micro-organismes. De plus, l'utilisation d'agents conservateurs, qui est essentielle lorsque la phase continue est aqueuse, peut être évitée, ou amoindrie, lorsque la phase continue est grasse. Les émulsions eau-dans-huile sont bien moins sensibles à une température basse que les émulsions huile-dans-eau. Enfin une phase continue huileuse permet de recouvrir la peau après application de l'émulsion eau-dans-huile, ce qui la protège de la déshydratation et contre des substances externes, en formant un film huileux persistant.

La demande de brevet français publiée sous le numéro 2 910 899 divulgue des formulations cosmétiques sous forme d'émulsions comportant un terpolymère réticulé d'AMPS, de N,N-diméthyl acrylamide et d'acrylate de lauryle tétraéthoxylé et un agent émulsionnant de type composition d'alcools gras et d'alkyl polyglucosides ou de type glycéride d'alcool gras.

La demande brevet européen publiée sous le numéro EP 1 426 436, divulgue des compositions de blanchiment comprenant un terpolymère réticulé d'AMPS, d'acrylamide et de Genapol™ LA070 méthacrylate

Le brevet publié sous le numéro US 5,746,945 divulgue des émulsions eau-dans-huile qui sont stabilisées par un système émulsifiant à deux composants, un copolymère polysiloxane polyalkyl polyéther et un dérivé de l'anhydride phtalique (un mono-amide). Elles sont préparées par ajout progressif de la phase aqueuse dans la phase huileuse, ces deux phases ayant été préalablement à 71-74°C (160 à 165°F).

La demande internationale publiée sous le numéro WO-97/40814 divulgue des émulsions eau-dans-huile destinées notamment à être utilisées pour imprégner des lingettes pour bébé et dans lesquelles les émulsionnants utilisés sont de type acide carboxylique, substitués par des hydrocarbures, ou des copolymères "block" ABA, impliquant des monomères tels que l'acide 12-hydroxy stéarique et l'éthylène glycol, ou un alkyldiméthicone copolyol.

La demande de brevet européen publiée sous le numéro EP 1.459 801 A2, divulgue la préparation d'émulsions eau-dans-huile comprenant, pour 100% de leurs masses, de 60% à 98% massique de phase aqueuse gélifiée grâce à la présence en son sein, d'un polyélectrolyte réticulé.

Cependant l'émulsion obtenue n'est pas suffisamment fluide et elle ne procure pas sur la peau un film huileux suffisamment persistant.

C'est pourquoi les inventeurs ont cherché à développer de nouvelles émulsions E/H qui ne présentent pas les inconvénients exposés ci-dessus, qui restent homogènes à température ambiante après un stockage d'au minimum trois mois ; dont la viscosité dynamique, mesurée à l'aide d'un viscosimètre de type Brookfield LV™ muni d'un mobile adapté et à une vitesse de 6 tour/minute, soit comprise à 20°C entre 500mPa.s et 40.000mPa.s ; que cette viscosité dynamique ne soit pas inférieure à 75% à la viscosité dynamique mesurée dans les mêmes conditions après 7 jours de stockage seulement ; dont le critère de consistance est évalué comme « liquide » avant son application sur la peau, l'évaluation étant réalisée en suivant le protocole décrit dans le paragraphe II-2.3.1 de la partie expérimentale du présent exposé ; et/ou dont la valeur du seuil d'écoulement, mesurée dans les conditions expérimentales telles que décrites dans le paragraphe II-2.3.2 de la partie expérimentale du présent exposé, est strictement inférieure à 40Pa.

C'est pourquoi, selon un premier aspect, l'invention a pour objet une composition (E₁) telle que définie à la revendication 1.

Par huile, on désigne, dans la définition de la composition (E₁) objet de la présente invention, un composé et/ou un mélange de composés insoluble dans l'eau, et liquide à 25°C, et plus particulièrement :
- Les huiles minérales telles que l'huile de paraffine, l'huile de vaseline, les isoparaffines ou les huiles blanches minérales ;
- Les huiles d'origine animale, telles que le squalène ou le squalane ;
- Les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;

- Les huiles végétales éthoxylées ;
- Les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées et
- Les huiles de silicone comme les diméthylpolysiloxanes, les méthylphénylpolysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Lorsque la composition (E₁) telle que définie ci-dessus comprend un cire, ctte dernière est plus particulièrement choisie parmi la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline; l'ozokérite; la cire de polyéthylène; les cires de silicone ; les cires végétales ; les alcools gras et les acides gras solides à température ambiante; les glycérides solides à température ambiante.

Par polyélectrolyte anionique réticulé (P), on désigne, dans la définition de la composition (E₁) objet de la présente invention, un polyélectrolyte anionique réticulé non linéaire, se présentant à l'état de réseau tridimensionnel insoluble dans l'eau, mais gonflable à l'eau et conduisant à l'obtention d'un gel chimique.

Par partiellement salifié ou totalement salifié, on signifie dans la définition du polyélectrolyte anionique réticulé (P) présent de la composition (E₁) telle que définie ci-dessus, que ledit acide 2-méthyl 2-[(1-oxo 2-propènyl) amino] 1-propanesulfonique est, partiellement ou totalement salifié, notamment sous forme de sel de métal alcalin, par exemple sous forme de sel de sodium ou de sel de potassium, ou sous forme de sel d'ammonium.

Par monomère neutre choisi parmi les N,N-dialkyl acrylamides, dans lesquels chacun des groupes alkyle comportent entre un et quatre atomes de carbones, on désigne, dans la définition de la composition (E₁) objet de la présente invention, plus particulièrement le N,N-diméthyl acrylamide, le N,N-diéthyl acrylamide, le N,N-dipropyl acrylamide, ou le N,N-diisopropyl acrylamide.

Selon un aspect particulier de la présente invention, la composition (E₁) telle que définie précédemment est caractérisée en ce qu'elle comprend pour 100% de sa masse :
- De 60% à 98% massique d'une phase aqueuse (A₁) comprenant pour 100% de sa masse, de 0,05% à 1,65% massique d'un polyélectrolyte anionique réticulé (P) issu de la polymérisation, en présence d'un agent de réticulation, de l'acide 2-méthyl 2-[(1-oxo 2-propènyl) amino] 1-propanesulfonique partiellement ou totalement salifié, avec un monomère neutre choisi parmi les N,N-dialkyl acrylamides, dans lesquels chacun des groupes alkyle comportent entre un et quatre atomes de carbones, et un monomère de formule (I) : 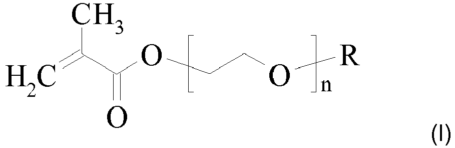 dans laquelle R représente un radical alkyle linéaire ou ramifié comportant de huit à vingt atomes de carbone et n représente un nombre entier supérieur ou égal à un et inférieur ou égal à vingt ;
- De 2% à 40% massique d'une phase grasse (A₂) comprenant pour 100 % de sa masse, de 1,25% à 50% massique d'un système émulsionnant (S) comprenant un ou plusieurs tensioactifs émulsionnants sélectionnés parmi les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés, les copolymères polyéthylèneglycol-alkylglycols ; de 50% à 98,75% massique d' une huile et optionnellement d'une cire.

Selon un aspect particulier de la présente invention, dans la composition (E₁) telle que définie ci-dessus, ledit polyélectrolyte anionique réticulé (P) comporte pour 100% molaire de ses monomères constitutifs, de 5% molaire à 95% molaire d'unités monomériques issues de l'acide 2-méthyl 2-[(1-oxo 2-propènyl) amino] 1-propanesulfonique partiellement ou totalement salifié, plus particulièrement de 10% molaire à 90% molaire, et tout particulièrement de 20% molaire à 80% molaire.

Selon un autre aspect particulier de la présente invention, dans la composition (E₁) telle que définie ci-dessus, ledit polyélectrolyte anionique réticulé (P) comporte pour 100% molaire de ses monomères constitutifs, de 4,9% molaire à 90% molaire d'unités monomériques issues d'au moins un monomère neutre choisi parmi les N,N-dialkyl acrylamides, dans lesquels chacun des groupes alkyle comportent entre un et quatre atomes de carbones, plus particulièrement de 9,5% molaire à 85% molaire, et tout particulièrement de 15% molaire à 75% molaire.

Selon un autre aspect particulier de la présente invention, dans la composition (E₁) objet de la présente invention, ledit polyélectrolyte anionique réticulé (P) tel que défini précédemment comporte pour 100% molaire de ses monomères constitutifs, de 0,1% molaire à 10% molaire et plus particulièrement de 0,5% molaire à 5% molaire, d'unités monomériques issues du monomère de formule (I).

L'invention a plus particulièrement pour objet une composition (E₁) telle que définie précédemment, caractérisée en ce que ledit polyélectrolyte anionique réticulé (P) comporte pour 100% molaire de ses monomères constitutifs :
- De 20% molaire à 80% molaire d'unités monomériques issues de lacide 2-méthyl 2-[(1-oxo 2-propènyl) amino] 1-propanesulfonique partiellement ou totalement salifiée ;
- De 15% molaire à 75% molaire d'unités monomériques issues d'au moins un monomère neutre choisi parmi les N,N-dialkyl acrylamides, dans lesquels chacun des groupes alkyle comportent entre un et quatre atomes de carbones ;
- De 0,5% à 5% molaire d'unités monomériques issues d'au moins monomère de formule (I).

Dans la formule (I) du monomère présent dans ledit polyélectrolyte anionique réticulé (P) compris dans la composition (E₁) objet de la présente invention, par radical alkyle linéaire ou ramifié comportant de huit à vingt atomes de carbone, on désigne plus particulièrement pour R :
- ou bien un radical dérivé des alcools primaires linéaires tels que par exemple, le radical octyle, décyle, undécyle, dodécyle, tridécyle, tétradécyle, pentadécyle, hexadécyle, heptadécyle, octadécyle, nonadécyle ou eicosyle ;
- ou bien un radical dérivé des alcools de Guerbet, qui sont des 1-alcanols ramifiés répondant à la formule générale :

   CH₃-(CH₂)ₚ-CH[CH₃-(CH₂)_{p-2]}-CH₂OH,

   dans laquelle p représente un nombre entier compris entre 2 et 9, tels que, par exemple, les radicaux 2-éthyl hexyle, 2-propyl heptyle, 2-butyl octyle, 2-pentyl nonyle, 2-hexyl décyle ou 2-octyl dodécyle ;
- ou bien un radical dérivé des isoalcanols répondant à la formule générale :

   CH₃-CH(CH₃)-(CH₂)ₘ-CH₂OH,
dans laquelle m représente un nombre entier compris entre 2 et 16, tels que, par exemple, les radicaux 4-méthyl pentyle, 5-méthyl hexyle, 6-méthyl heptyle, 15-méthyl pendadécyle ou 16-méthyl heptadécyle, soit les radicaux 2-hexyl octyle, 2-octyl décyle ou 2-hexyl dodécyle.

Selon un autre aspect particulier, la composition (E₁) telle que définie précédemment, est caractérisée en ce que ledit monomère neutre est le N,N-diméthyl acrylamide.

Selon un autre aspect particulier, l'invention a pour objet une composition (E₁) telle que définie précédemment, caractérisée en ce que dans ledit polyélectrolyte anionique réticulé (P) et pour ledit monomère de formule (I) telle que définie précédemment, R représente un radical alkyle comportant de 12 à 18 atomes de carbone.

Selon un aspect encore plus particulier, l'invention a pour objet une composition (E₁) telle que définie précédemment, caractérisée en ce que dans ledit polyélectrolyte anionique réticulé (P) et pour ledit monomère de formule (I) telle que définie précédemment, R représente un radical alkyle choisi parmi les éléments du groupe constitué par le radical dodécyle, tridécyle, tétradécyle, hexadécyle, octadécyle.

Selon un autre aspect particulier, l'invention a pour objet une composition (E₁) telle que définie précédemment, caractérisée en ce que dans ledit polyélectrolyte anionique réticulé (P) et pour ledit monomère de formule (I) telle que définie précédemment, n représente un nombre entier supérieur ou égal à 3 et inférieur ou égal à 20.

Selon un aspect encore plus particulier, la composition (E₁) telle que définie précédemment, est caractérisée en ce que ledit monomère de formule (I) est le méthacrylate de lauryle tétraéthoxylé.

Selon un autre aspect encore plus particulier, l'invention a pour objet une composition (E₁) telle que définie précédemment, caractérisée en ce que dans ledit polyélectrolyte anionique réticulé (P) tel que défini précédemment, ledit monomère de formule (I) est le méthacrylate de stéaryle eicosaéthoxylé.

Selon un autre aspect particulier, l'invention a pour objet une composition (E₁) telle que définie précédemment, caractérisée en ce que dans ledit polyélectrolyte anionique réticulé (P) tel que défini précédemment est réticulé avec un composé diéthylénique ou polyéthylènique dans la proportion molaire exprimée par rapport aux monomères mis en oeuvre, est de 0,005% à 1% molaire, plus particulièrement de 0,01% à 0,5% molaire et tout particulièrement de 0,01% à 0,25% molaire. L'agent de réticulation est plus particulièrement choisi parmi le diméthacrylate d'éthylèneglycol, le tétraallyloxyéthane, le diacrylate d'éthylèneglycol, le diallyl urée, le triallyl amine, le triméthylol propanetriacrylate ou le méthylène-bis(acrylamide) ou un mélange de ces composés.

Le polyélectrolyte anionique réticulé (P) mis en oeuvre dans la composition (E₁) objet de la présente invention peut également comprendre divers additifs, tels que des agents complexants, des agents de transfert ou des agents limiteurs de chaîne. Les agents de transfert ou limiteurs de chaîne sont plus particulièrement choisi parmi le groupe constitué par l'hypophosphite de sodium, des alcools de faibles poids moléculaires par exemple le méthanol, l'éthanol, le propanol-1, l'isopropanol, le butanol, des thiols, par exemple le 2-mercapto éthanol, des agents de transfert comprenant une fonction sulfate, par exemple le methallylsulfonate de sodium, ou des mélanges desdits agents de transfert. Les agents de transfert ou limiteurs de chaînes sont plus particulièrement utilisés dans des proportions molaires, exprimées par rapport au nombre total de moles de monomères mis en oeuvre, de 0,001% à 1% molaire, plus particulièrement de 0,001% à 0,5% molaire, et tout particulièrement de 0,001% à 0,1% molaire.

Selon un aspect particulier, l'invention a pour objet une composition (E₁) telle que définie précédemment, caractérisée en ce que ledit polyélectrolyte anionique réticulé (P) tel que défini précédemment est choisi parmi les terpolymères de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate ou les terpolymères de l'acide 2-méthyl 2- [(1-oxo 2-propènyl) amino] 1-propanesulfonique partiellement salifié sous forme de sel d'ammonium, du N,N-diméthyl acrylamide et du méthacrylate de stéaryle eicosaéthoxylé, réticulé au triméthylol propanetriacrylate.

Selon un aspect plus particulier, l'invention a pour objet une composition (E₁) telle que définie précédemment, caractérisée en ce que ledit polyélectrolyte anionique réticulé (P) est un terpolymère de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate.

Selon un autre aspect plus particulier, l'invention a pour objet une composition (E₁) telle que définie précédemment, caractérisée en ce que ledit polyélectrolyte anionique réticulé (P) comporte pour 100% molaire :
- De 60% molaire à 80% molaire d'unités monomériques issues de l'acide 2-méthyl 2-[(1-oxo 2-propènyl) amino] 1-propanesulfonique partiellement salifié sous forme d'ammonium,
- De 15% molaire à 39,5% molaire d'unités monomériques issues du N,N-diméthyl acrylamide, et
- De 0,5% molaire à 5% molaire d'unités monomériques issues du méthacrylate de lauryle tétraéthoxylé.

Selon un autre aspect particulier, l'invention a pour objet une composition (E₁) telle que définie précédemment caractérisée en ce que sa viscosité dynamique de ladite composition (E1), mesurée à une température de 20°C et à l'aide d'un viscosimètre Brookfield de type LVT à une vitesse de 6 tours/minute, est supérieure ou égale à 500 mPa.s et inférieure ou égale à 40 000 mPa.s.

L'invention a pour aussi pour objet l'utilisation de la composition (E₁) telle que définie précédemment, pour le nettoyage, pour la protection et/ou pour le soin de la peau, des cheveux, du cuir chevelu ou des muqueuses.

La composition (E₁) objet de la présente invention telle que définie précédemment est destinée à un usage topique, et peut être incorporée dans tout type de formulation cosmétique, dermocosmétique, dermopharmaceutique ou pharmaceutique destinée à un usage topique, ou bien encore dans tout type de support destiné à être mis en contact avec la peau (papier, lingette, textile, dispositif transdermique, etc.).

L'expression "à usage topique" signifie que la composition (E₁) est mise en oeuvre par application sur la peau, les cheveux, le cuir chevelu ou les muqueuses, qu'il s'agisse d'une application directe dans le cas d'une formulation cosmétique, dermocosmétique, dermopharmaceutique ou pharmaceutique ou d'une application indirecte par exemple dans le cas d'un produit d'hygiène corporelle, de soin ou de protection de la peau, se présentant sous la forme d'un article en textile, par exemple une lingette, ou en papier, par exemple un papier à usage sanitaire.

La composition (E₁) objet de la présente invention peut être conditionnée sous forme pressurisée dans un dispositif aérosol ou dans un dispositif de type « flacon pompe », dans un dispositif muni d'une paroi ajourée par exemple une grille, dans un dispositif muni d'un applicateur a billes (dit"roll-on).

La composition (E₁) objet de la présente invention peut être utilisée comme laits nettoyants ou démaquillants, comme lotions nettoyantes ou démaquillantes, comme gels moussants pour le visage ou pour le corps, comme shampooing pour le nettoyage des cheveux et/ou du cuir chevelu, comme après-shampooing pour le traitement des cheveux et/ou du cuir chevelu, comme bain moussant, comme crème, comme lait ou comme lotion pour le soin ou pour la protection du visage, des mains et du corps, par exemple comme agent protecteur des rayonnements solaires, comme agent auto-bronzant, comme agent anti-âge, comme agent antirides, comme agent apaisant, comme agent hydratant.

La composition (E₁) objet de la présente invention peut en outre comporter des excipients et/ou des principes actifs habituellement mis en oeuvre dans le domaine des formulations à usage topique, en particulier cosmétiques, dermocosmétiques, pharmaceutiques ou dermo-pharmaceutique.

C'est pourquoi, selon un autre mode particulier, l'invention a pour objet une composition (E₁) telle que définie précédemment, caractérisée en ce qu'elle comprend en outre, un ou plusieurs composés auxiliaires chois parmi les tensioactifs moussants et/ou détergents, les tensioactifs épaississants et/ou gélifiants, les agents épaississants et/ou gélifiants, les agents stabilisants, les composés filmogènes, les solvants et cosolvants, les agents hydrotropes, les agents plastifiants, les agents émulsionnants et co-émulsionnants, les agents opacificants, les agents nacrants, les agents surgraissants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents, éventuellement présents dans la composition (E₁) objet de la présente invention, on peut citer les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents que l'on peut associer à la composition (E₁) objet de la présente invention, on peut citer les sels de métaux alcalins, les sels de métaux alcalino-terreux, les sels d'ammonium, les sels d'amines, les sels d'amino alcools d'alkyléthers sulfates, d'alkyl sulfates, d'alkylamidoéther sulfates, d'alkylarylpolyéther sulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfo-acétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, d'acides gras.

Parmi les tensioactifs amphotères moussants et/ou détergents éventuellement présents dans la composition (E₁) objet de la présente invention, on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents éventuellement présents dans la composition (E₁) objet de la présente invention, on peut citer particulièrement les dérivés d'ammoniums quaternaires.

Parmi les tensioactifs non ioniques moussants et/ou détergents éventuellement présents dans la composition (E₁) objet de la présente invention, on peut citer plus particulièrement les alkylpolyglycosides comportant un radical aliphatique, linéaire ou ramifié, saturé ou insaturé, et comportant de 8 à 12 atomes de carbone ; les dérivés d'huile de ricin, les polysorbates, les amides de coprah, les N-alkylamines.

Comme exemples de tensioactifs épaississants et/ou gélifiants éventuellement présents dans la composition (E₁) objet de la présente invention, on peut citer :
- les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ;
- les esters gras alcoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141 ;
- les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples de tensioactifs émulsionnants éventuellement présents dans la composition (E₁) objet de la présente invention, on peut citer des tensioactifs non ioniques, des tensioactifs anioniques, des tensioactifs cationiques.

Comme exemples de tensioactifs non ioniques émulsionnants éventuellement présents dans la composition (E₁) objet de la présente invention, on peut citer les esters d'acides gras et de sorbitol, par exemple les produits commercialisés sous les appellations MON-TANE™80 et MONTANE™85 et MONTANE™60; l'huile de ricin éthoxylée et l'huile de ricin hydrogénée éthoxylée, par exemple le produit commercialisé sous la dénomination SIMUL-SOL™ 989; les compositions comprenant du stéarate de glycérol et de d'acide stéarique poly(éthoxylé) avec entre 5 moles et 150 moles d'oxyde d'éthylène, par exemple la composition comprenant de l'acide stéarique (éthoxylé) à 135 moles d'oxyde d'éthylène et du stéarate de glycérol commercialisée sous l'appellation SIMULSOL™ 165 ; les esters de sorbitan éthoxylés, par exemple les produits commercialisés sous la dénomination MONTANOX™ ; les esters de mannitan ; les esters de mannitan éthoxylés ; les esters de sucrose ; les esters de méthylglucoside.

Comme exemples de tensioactifs anioniques émulsionnants éventuellement présents dans la composition (E₁) objet de la présente invention, on peut citer le décylphosphate, le cétylphosphate commercialisé sous l'appellation AMPHISOL™, le glycéryl stéarate citrate ; le cétéarylsulfate ; la composition arachidyl/béhényl phosphates et arachidyl/béhényl alcools commercialisée sous l'appellation SENSANOV™WR; les savons par exemple le stéarate de sodium ou le stéarate de triéthanolammonium, les dérivés N-acylés d'acides aminés salifiés comme par exemple le stéaroyl glutamate.

Comme exemples de tensioactifs cationiques émulsionnants éventuellement présents dans la composition (E₁) objet de la présente invention, on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants éventuellement présents dans la composition (E₁) objet de la présente invention, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture éventuellement présents dans la composition (E₁) objet de la présente invention, on peut citer des dérivés N-acylés d'acides aminés, par exemple la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV 14, les fibres de cellulose, les fibres de coton, les fibres de chitosane, le talc, la séricite, le mica.

Comme exemples de solvants et de co-solvants éventuellement présents dans la composition (E₁) objet de la présente invention, on peut citer l'eau, les solvants organiques par exemple le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques.

Comme exemples de principes actifs éventuellement présents dans la composition (E₁) objet de la présente invention, il y a :
- Les vitamines et leurs dérives, par exemple, le rétinol (vitamine A) et ses esters (palmitate de rétinyle par exemple), l'acide ascorbique (vitamine C) et ses esters, les dérives de sucre de l'acide ascorbique (par exemple l'ascorbyl glucoside), le tocophérol (vitamine E) et ses esters (par exemple l'acétate de tocophérol), les vitamines B3 ou B10 (niacinamide et ses dérivés) ;
- Les composés ayant une action éclaircissante ou dépigmentante de la peau, par exemple le SEPIWHITE™MSH, l'arbutine, l'acide kojique, l'hydroquinone, le VEGEWHITE™, la GATULINE™, le SYNERLIGHT™, le BIOWHITE™, le PHYTOLIGHT™, le DERMA-LIGHT™, la CLARISKIN™, le MELASLOW™, le DERMAWHITE™, l'ETHIOLINE, le MELA-REST™, le GIGAWHITE™, l'ALBATINE™, le LUMISKIN™ ;
- Les composés ayant une action apaisante comme le SEPICALM™ S, l'allantoïne et le bisabolol ;
- Les agents anti-inflammatoires ;
- Les composés montrant une action hydratante par exemple l'urée, les hydroxyurées, le glycérol, les polyglycérols, le glycérolglucoside, le diglycérolglucoside, les polyglycérylglucosides ;
- Les composés montrant une action amincissante ou lipolytique comme la caféine ou ses dérivés, l'ADIPOSLIM™, l'ADIPOLESS™ ;
- Les protéines N-acylées ; les peptides N-acylés par exemple le MATRIXIL™ ; les acides aminés N-acylés ; les hydrolysâts partiels de protéines N-acylés ; les acides aminés ; les peptides ; les hydrolysâts totaux de protéines ;
- Les extraits végétaux riches en tanins en polyphénols et/ ou en isoflavones, par exemple les extraits de raisin, les extraits de pin, les extraits de vin, les extraits d'olives ; les extraits de soja, par exemple la Raffermine™ ; les extraits de blé par exemple la TENSINE™ ou la GLIADINE™ ; les extraits végétaux riches en terpènes ; les extraits d'algues d'eau douce ou marines ; les extraits marins en général comme les coraux ;
- Les cires essentielles ; les extraits bactériens ; les céramides ou les phospholipides ;
- Les composés ayant une action antimicrobienne ou une action purifiante, par exemple le LIPACIDE™ C8G, le LIPACIDE™ UG, le SEPICONTROL™ A5 ; l'OCTOPI-ROX™ ou le SENSIVA™ SC50 ;
- Les composés ayant une propriété énergisante ou stimulante comme le Physiogényl™, le panthénol et ses dérivés comme le SEPICAP™ MP ;
- Les actifs anti-âge comme le SEPILIFT™ DPHP, le LIPACIDE™ PVB, le SEPIVI-NOL™, le SEPIVITAL™, le MANOLIVA™, le PHYTO-AGE™, le TIMECODE™ ; le SURVI-CODE™ ;
- Les actifs anti-photo vieillissement ; les actifs protecteurs de l'intégrité de la jonction dermo-épidermique ;
- Les actifs augmentant la synthèse des composants de la matrice extracellulaire par exemple le collagène, les élastines, les glycosaminoglycanes ;
- Les actifs agissant favorablement sur la communication cellulaire chimique comme les cytokines ou physiques comme les intégrines ;
- Les actifs créant une sensation de « chauffe » sur la peau comme les activateurs de la microcirculation cutanée (par exemple les dérivés de l'acide nicotinique) ou des produits créant une sensation de « fraîcheur » sur la peau (par exemple le menthol et des dérivés) ;
   Les actifs améliorant la microcirculation cutanée, par exemple les veinotoniques ; les actifs drainants ; les actifs à visée décongestionnante par exemple les extraits de ginko biloba, de lierre, de marron d'inde, de bambou, de ruscus, de petit houx, de *centalla asiatica,* de fucus, de romarin, de saule ;
- Les agents de bronzage ou de brunissement de la peau, par exemple la dihydroxyacétone, l'isatine, l'alloxane, la ninhydrine, le glycéraldéhyde, l'aldéhyde mésotartrique, la glutaraldéhyde, l'érythrulose.

Comme exemples d'agents épaississants et/ou gélifiants éventuellement présents dans la composition (E₁) objet de la présente invention, on peut citer les homopolymères ou copolymères de l'acide acrylique ou de dérivés de l'acide acrylique, les homopolymères ou copolymères de l'acrylamide, les homopolymères ou copolymères de l'acide acrylamidométhyl propanesulfonique, de monomère vinylique, de chlorure de triméthylaminoéthylacrylate, les hydrocolloïdes d'origine végétale ou biosynthétique, par exemple la gomme de xanthane, la gomme de karaya, les carraghénates, les alginates ; les galactomannanes par exemple la gomme de tara, la gomme de guar, la gomme de fenugrec, la gomme de caroube, la gomme de casse ; les silicates ; la cellulose et ses dérivés ; l'amidon et ses dérivés hydrophiles ; les polyuréthanes.

Comme exemples d'agents déodorants éventuellement présents dans la composition (E₁) objet de la présente invention, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2-décanediol ; le 1,3-propanediol ; l'acide salicylique ; le bicarbonate de sodium ; les cyclodextrines ; les zéolithes métalliques ; le Triclosan™ ; le bromohydrate d'aluinium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples de filtres solaires éventuellement présents dans la composition (E₁) objet de la présente invention, on peut citer tous ceux figurant dans la directive cosmétique 76/768/CEE modifiée annexe VII.

Parmi les filtres organiques solaires éventuellement présents dans la composition (E₁) objet de la présente invention, on peut citer :
- Les dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Les dérivés de l'acide anthranilique comme l'homomenthyl-N-acétyl anthranilate ;
- Les dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ;
- Les dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-□α-cyano-β-phényle, le cinnamate de diparaméthoxy mono-2-éthylhexanoyl de glycéryle ;
- Les dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-carboxylate, la 2-hydroxy-4-n-octyloxybenzophénone, la 4-hydroxy-3-carboxybenzophénone ; le 3-(4'-méthylbenzylidène)-d,l-camphre, le 3-(benzylidène)-d,l-camphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ;
- Les dérivés de la triazine comme l'hydroxyphényl triazine, l'(éthylhexyloxyhydroxyphényl) (4-méthoxy phényl) triazine, le 2,4,6-trianillino (p-carbo-2'-éthylhexyl-1'-oxy)-1,3,5-triazine, le benzoate de 4,4-((6-(((1,1-diméthyléthyl) amino)carbonyl)phenyl)amino)-1,3,5-triazine-2,4-diyl diimino) bis-(2-éthylhexyl), le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-t-butylbenzoylméthane ; la 5-(3,3-diméthyl-2-norbornylidène)-3-pentan-2-one ;
- Les dérivés du diphénylacrylate comme le 2-éthylhexyl-2-cyano-3,3-diphényl-2-propènoate, l'éthyl-2-cyano-3,3-diphényl-2-propènoate ;
- Les polysiloxanes comme le malonate de benzylidène siloxane.

Parmi les filtres inorganiques solaires, également appelés "écrans minéraux", éventuellement présents dans la composition (E₁) objet de la présente invention, on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

L'invention a aussi pour objet l'utilisation de la composition (E₁) telle que définie précédemment, pour le traitement cosmétique de la peau, des cheveux et/ou des muqueuses et plus particulièrement pour le nettoyage, pour la protection et/ou pour le soin de la peau, des cheveux, du cuir chevelu ou des muqueuses.

L'invention a aussi pour objet un procédé de préparation de la composition (E₁) telle que définie précédemment, comprenant les étapes suivantes:
Une étape a) de préparation de la phase grasse (A₂) en mélangeant l'ensemble des éléments la constituant dans les proportions souhaitées. Cette étape de mélange est généralement conduite à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C ; elle est réalisée sous agitation mécanique à une vitesse modérée supérieure ou égale à 50 tours/minute et inférieure ou égale à 100 tours/minute ;
Une étape b) de préparation de la phase aqueuse (A₁) de l'ensemble des éléments la constituant dans les proportions souhaitées. Cette étape de mélange est généralement conduite à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C ; elle est réalisée sous agitation mécanique à une vitesse modérée supérieure ou égale à 500 tours/minute et inférieure ou égale à 3 000 tours/minute. De façon particulière, la phase aqueuse (A₁) obtenue à l'issue de l'étape b), présente une viscosité dynamique, mesurée à 20°C par l'intermédiaire d'un viscosimètre de type Brookfield LV à une vitesse de 6 tours/minute, supérieure ou égale à 200 mPa.s et inférieure ou égale à 40 000 mPa.s, plus particulièrement supérieure ou égale à 1 000 mPa.s et inférieure ou égale à 40 000 mPa.s, et encore plus particulièrement supérieure ou égale à 2 000 mPa.s et inférieure ou égale à 40 000 mPa.s ;
   - Une étape c) au cours de la quelle la phase grasse (A₂) est ajoutée sur la phase aqueuse (A₁) à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C, sous agitation mécanique à une vitesse modérée supérieure ou égale à 50 tours/minute et inférieure ou égale à 400 tours/minute, de façon à obtenir la composition (E₁).

### I) - Préparation de polyélectrolytes anioniques réticulés.

### I-1 Terpolymère de 2-méthyl 2-[(1-oxo 2-propènyl) amino] 1-propanesulfonate d'ammonium, de N,N-diméthyl acrylamide et de méthacrylate de lauryle tétraéthoxylé [AMPSNH₄/DMAM/MAL(4OE) 77,4/19,2/3,4 molaire], réticulé au propanetriacrylate de triméthylol (TMPTA).

On charge dans un réacteur maintenu à 25°C sous agitation, 592g d'une solution aqueuse à 15% massique de 2-méthyl 2-[(1-oxo 2-propènyl) amino] 1-propanesulfonate d'ammonium (AMPSNH₄) dans un mélange tert-butanol/eau (97,5/2,5 en volume), 10,1g de N,N-diméthyl acrylamide (DMAM), 4,2g de méthacrylate de lauryle tétra-éthoxylé [MAL(4OE)] et 0,75g de TMPTA. Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C. Après refroidissement, la poudre qui s'est formée en cours de polymérisation, est filtrée et séchée pour obtenir le produit désiré, dénommé par la suite : "Polyélectrolyte (PA₁)".

### I-2 Terpolymère de 2-méthyl 2-[(1-oxo 2-propènyl) amino] 1-propanesulfonate d'ammonium et de N,N-diméthyl acrylamide [AMPSNH₄/DMA 80/20 molaire], réticulé au propanetriacrylate de triméthylol (TMPTA).

On charge dans un réacteur maintenu à 25°C sous agitation, 592g d'une solution aqueuse à 15% massique d'AMPSNH₄ dans un mélange tert-butanol/eau (97,5/2,5 en volume), 10,1g de DMAM et 0,75g de TMPTA. Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C. Après refroidissement, la poudre qui s'est formée en cours de polymérisation, est filtrée et séchée pour obtenir le produit désiré, dénommé par la suite : "Polyélectrolyte (PA₂)".

### II) - Préparation et évaluation d'émulsions eau-dans-huile selon l'invention et d'émulsions eau-dans-huile comparatives.

### II-1 Préparation des émulsions eau-dans-huile

On prépare quatre émulsions eau-dans-huile selon l'invention, notées (F₁) à (F₄), et six émulsions eau-dans-huile selon l'état de la technique, notées (F'₁) à (F'₆), dont les proportions massiques en leurs constituants sont consignées dans le tableau 1 ci-dessous les teneurs massiques des polyélectrolytes étant indiquées en pourcentage de matière sèche polymérique, en mettant en oeuvre le procédé suivant :
Les constituants de la phase grasse sont introduits successivement dans un bêcher, et mélangés à une température de 20°C, à l'aide d'un agitateur mécanique muni d'un mobile d'agitation de type hélice, à une vitesse de 100 tours/minute. La glycérine et l'eau sont mélangées à température ambiante dans un bêcher à l'aide d'un agitateur mécanique à une vitesse de 2 000 tours/minute et l'agent épaississant est alors ajouté progressivement. L'agitation est maintenue pendant une durée permettant d'atteindre une phase aqueuse se présentant sous la forme d'un gel homogène. La phase grasse est ajoutée en une seule fois sur le gel aqueux, à température ambiante et à une vitesse d'agitation modérée (75 à 300 tours/minute) avec un agitateur équipé d'un mobile de type ancre. Cette agitation est alors maintenue pendant dix minutes et aucune étape de refroidissement n'est nécessaire. Les émulsions (F'₃) et (F'₄) ainsi préparées sont représentatives de l'enseignement technique de la demande de brevet européen publiée sous le numéro EP 1.459 801 A2.

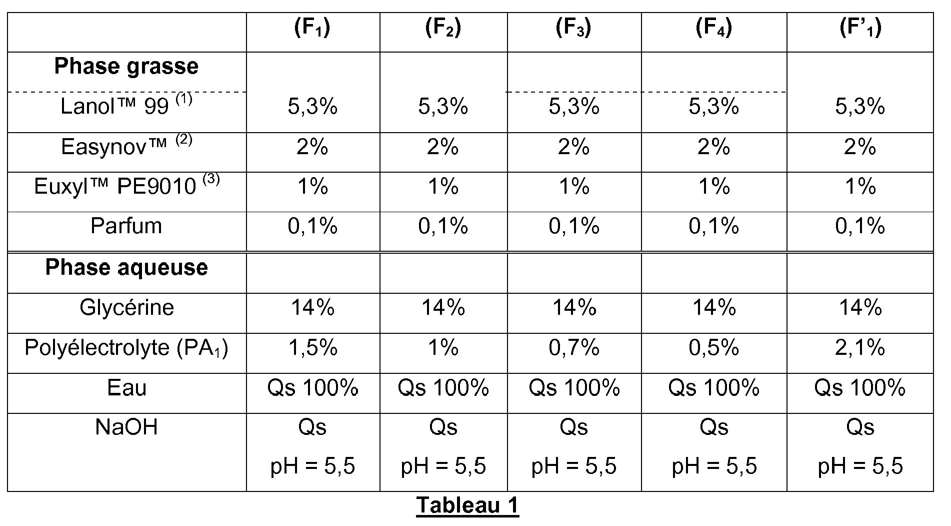

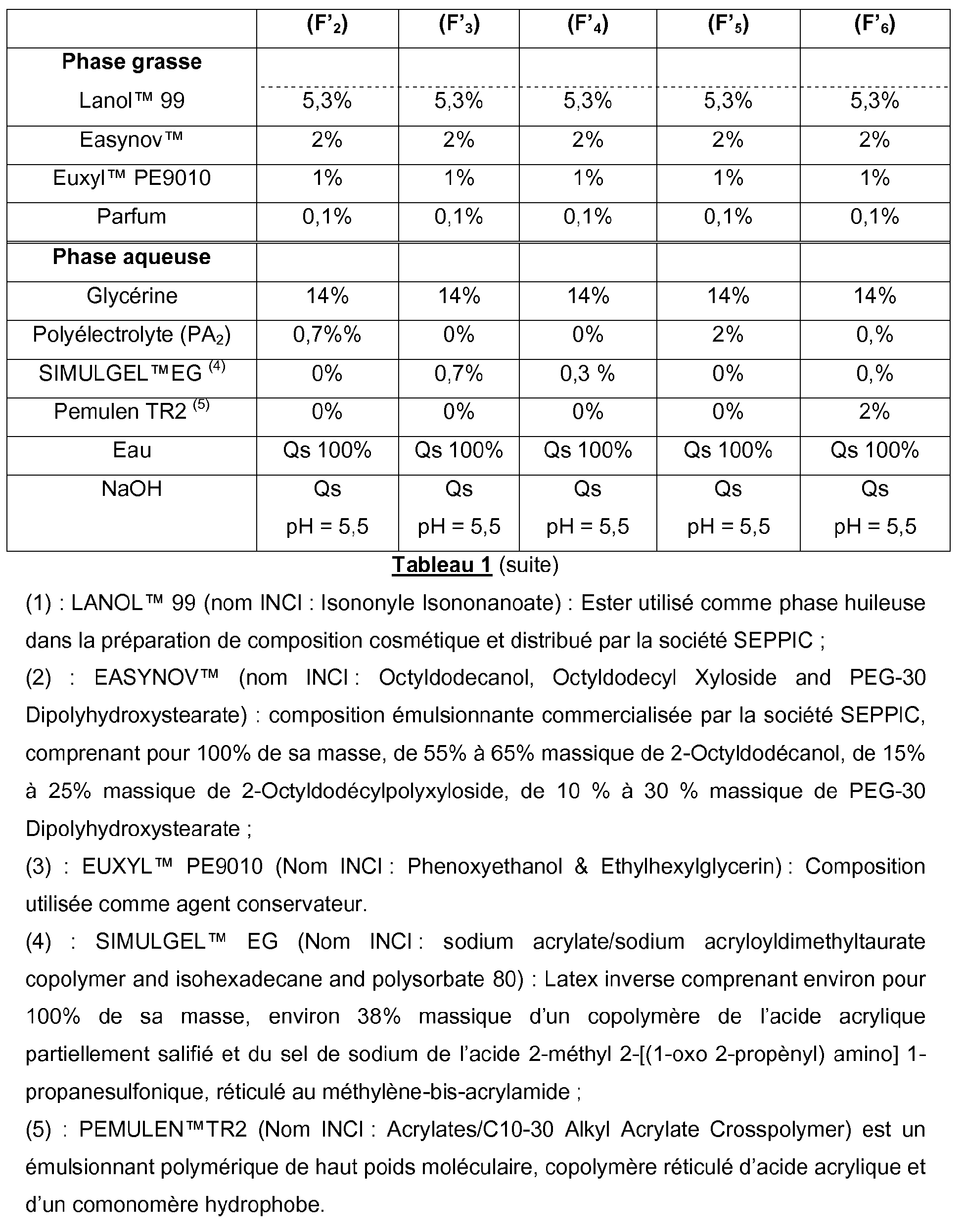

### II-2 Mise en évidence des propriétés des émulsions eau-dans-huile (F₁) à (F₄) selon l'invention et des émulsions eau-dans-huile (F'₁) à (F'₆) selon l'état de la technique.

### II-2.1 Caractérisation de l'aspect et de la viscosité des émulsions eau-dans-huile (F₁) à (F₄) selon l'invention et des émulsions eau-dans-huile (F'₁) à (F'₆) selon l'état de la technique.

Les émulsions (F₁) à (F₄), et (F'₁) à (F'₆) obtenues selon le procédé précédemment décrit, sont ensuite conservées dans une enceinte climatique isolée et régulée à une température de 20°C pendant 7 jours. A l'issue de cette période de 7 jours, l'aspect (ASP) de chaque émulsion préparée est observé et la viscosité dynamique **(µ)** de chaque émulsion est mesurée (en mPas). Les émulsions sont ensuite replacées et conservées dans la même enceinte climatique isolée et régulée à une température de 20°C jusqu'à 3 mois. Après une période d'un mois, chaque émulsion est sortie de l'enceinte climatique pour en observer l'aspect et pour en mesurer la viscosité dynamique à 20°C, au moyen d'un viscosimètre à 20°C (Brookfield LVT, vitesse 6).

### II-2.2 Caractérisation du sens des émulsions (F₁) à (F₄) selon l'invention et des émulsions (F'₁) à (F'₆) selon l'état de la technique.

La conductivité **(σ)** des émulsions (F₁) à (F₄) selon l'invention et des émulsions (F'₁) à (F'₆) est mesurée à 20°C, après une durée de stockage desdites émulsions de un jour dans une enceinte climatique isolée et régulée à une température de 20°C, au moyen d'un conductimètre de marque LF 196™ de la société WTW muni d'une électrode Tétracon™ 96.

Si pour une émulsion donnée, **(σ)** est inférieure ou égale à 0,5 µS.cm⁻¹, on considère que l'émulsion n'est pas conductrice et que par conséquent la phase externe n'est pas la phase aqueuse mais la phase huileuse ; une telle émulsion pour laquelle **(σ)** est inférieure ou égale à 0,5 µS.cm⁻¹ est donc une émulsion eau-dans-huile.

### II-2.3 Evaluation de la consistance d'une composition.

Par consistance **(γ)** d'une composition, on entend la résistance à l'écoulement de ladite composition, pouvant être caractérisée par des observations empiriques, telle que l'observation visuelle de l'écoulement de la composition et/ou par la mesure d'un seuil d'écoulement à l'aide d'un rhéomètre de type cône-plan et relié à un logiciel de traitement des données.

### II-2.3.1 Evaluation de la consistance de la composition par observation visuelle

### a) Principe de la méthode

La consistance peut être évaluée par une observation visuelle de l'écoulement de la composition évaluée qui est contenue dans un flacon suite à une rotation de 180° dudit flacon.

### b) Protocole expérimental

Une quantité de 50 grammes de la composition testée est introduite dans un flacon en verre transparent de 100 mL, qui est fermé à l'aide d'un bouchon à vis. L'expérimentateur dûment entrainé et expérimenté retourne à 180° le flacon contenant la composition testée pendant une durée de 3 secondes et note son observation. Il fait alors subir une nouvelle rotation de 180° au flacon contenant la composition testée.

### c) Expression des résultats

- Si pendant la durée de 3 secondes, pendant laquelle le flacon contenant est retourné de 180°, la composition testée s'écoule spontanément, ou si pendant la durée de 3 secondes, pendant laquelle le flacon contenant est retourné de 180°, la composition testée ne s'écoule pas spontanément mais s'écoule à la suite de trois impulsions manuelles données sur le fond du flacon par l'expérimentateur, la composition à usage est qualifiée de "liquide".
- Si pendant la durée de 3 secondes, pendant laquelle le flacon contenant est retourné de 180°, la composition testée ne s'écoule pas spontanément et si elle ne s'écoule pas à la suite de trois impulsions manuelles données sur le fond du flacon par l'expérimentateur, la composition testée est qualifiée de "compacte".

### II-2.3.2 Evaluation de la consistance d'une composition par la mesure d'un seuil d'écoulement

### a) Principe de la méthode

La consistance peut être évaluée par la mesure d'un seuil d'écoulement (SE), mise en oeuvre à l'aide d'un rhéomètre de modèle AR2000 commercialisé par la société TA Instruments, et muni d'un plan Peltier pour contôler la température et d'un cône, relié à un logiciel de traitement des données.

### b) Protocole expérimental

La composition testée est déposée sur le plan 24 heures après sa fabrication.

Le rhéomètre de modèle AR2000 est constitué d'un système cône plan avec palier à air, le plan muni d'un système Peltier pour contrôler la température de mesure. Le cône est en aluminium, il mesure 6 centimètres de diamètre, son angle est de 1°. L'échantillon positionné dans l'entrefer du plan et du cône est cisaillé en mode « oscillation » et plus particulièrement en mode balayage en contrainte, à une température 25°C, à une fréquence d'oscillation de 1 Hz. Le capteur de force mesure, en continu, le déphasage de résistance du produit face à la contrainte sollicitée dans l'entrefer. L'exploitation de ces mesures par le logiciel permet de déduire la viscoélasticité de l'échantillon, à savoir la valeur G' (en Pa) de la composante solide élastique et la valuer G" (en Pa) de la composante liquide visqueux. Lorsque la valeur de G" est supérieure à la valeur de G', l'échantillon se comporte comme un liquide, et il s'écoule. Lors d'un balayage en contrainte croissante, de 0,1 à 100 Pa, l'échantillon se comporte comme un solide aux faibles contraintes, puis comme un liquide aux plus fortes contraintes, au delà d'un seuil nommé seuil d'écoulement. La valeur de la contrainte seuil, ou seuil d'écoulement (en Pa) s'extrapole de la courbe de G', comme point de croisement des tangentes au point d'inflexion. La précision est de l'ordre de 1 Pa.

### c) Expression des résultats

- Si la valeur de seuil d'écoulement, mesurée dans les conditions expérimentales telles que décrites ci-dessus, est supérieure ou égale à une valeur de 40 Pa, la composition testée est considérée comme "compacte".
- Si (SE) < 40Pa, la composition testée est considérée comme "liquide".

### II-2.4 Résultats obtenus pour les émulsions eau-dans-huile (F₁) à (F₄) selon l'invention et pour les émulsions eau-dans-huile (F'₁) à (F'₆) selon l'état de la technique.

Les méthodes d'évaluations décrites aux paragraphes II-2.1 et II-2.2 ont été appliquées aux émulsions eau-dans-huile (F₁) à (F₄) selon l'invention et aux émulsions eau-dans-huile (F'₁) à (F'₆) selon l'état de la technique.
Les résultats obtenus sont consignés dans la tableau 2 ci-dessous.

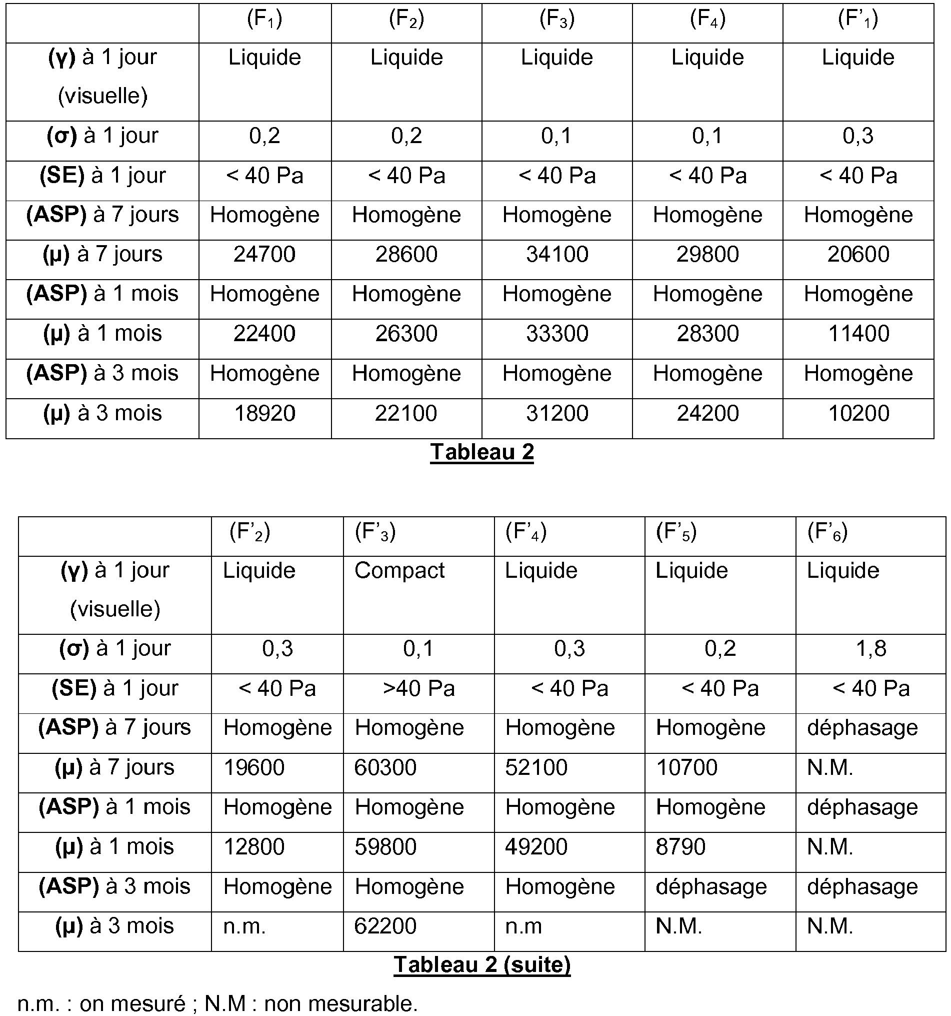

De plus il a été observé que lorsque les formulations eau-dans-huile (F₁), (F₂), (F₃) et (F₄) selon l'invention, sont appliquées sur la peau, elles se caractérisent par la formation d'un film huileux sur ladite peau, persistant au cours du temps pendant la durée d'étalement.

### II-2.5 Analyse des résultats

Les émulsions eau-dans-huile (F₁), (F₂), (F₃) et (F₄) selon l'invention, se caractérisent donc :
- Par une stabilité de leur forme eau-dans-huile après 3 mois de stockage à une température de 20°C ; l'aspect observé après cette période de stockage est toujours homogène ;
- Par une consistance jugée « Fluide » après un jour à 20°C selon la méthode d'observation visuelle décrite au paragraphe II-2.3.1 du présent exposé ;
- Par une valeur de seuil d'écoulement, mesurée dans les conditions expérimentales telles que décrites dans le paragraphe II-2.3.2 de la partie expérimentale du présent exposé, strictement inférieure à une valeur de 40 Pa,
- Par des valeurs de viscosités dynamiques mesurées après 7 jours de stockage à 20°C, au moyen d'un viscosimètre de modèle Brookfield LV à 20°C et à une vitesse de 6 tours/minute, comprises entre 24 700 mPa.s (pour (F₁)) et 34 100 mPa.s (pour (F₃)) ;
- Par des baisses limitées de viscosité dynamique après un stockage pendant trois mois à 20°C, puisque les diminutions de la valeur desdites viscosités dynamiques mesurées après 3 mois de stockage à 20°C (Brookfield LV, 6 tours/minute à 20°C) ne sont pas supérieure à plus de 25% de la valeur de la viscosité dynamique mesurée après 7 jours de stockage dans les mêmes conditions. Ainsi, pour un stockage à 20°C, la diminution de la valeur de la viscosité dynamique entre le 7^{ème} jour de stockage et la fin du 3^{ème} mois de stockage est de 15,5% pour (F₁), 22,7% pour (F₂), 8,5% pour (F₃) et 18,8% (F₄).

L'émulsion (F'₁) se distingue des émulsions selon l'invention par une quantité de polyélectrolyte anionique réticulé « Polyélectrolyte (PA₁) » supérieure à 2% pour 100% de la masse de l'émulsion, soit supérieure à 1,65% pour 100% de la masse de la phase aqueuse, ce qui entraine de façon inattendue une baisse de 50,5% de la viscosité dynamique de ladite émulsion (F'₁) entre le 7^{ème} jour et la fin du 3^{ème} mois de stockage à 20°C.

Lorsque des émulsions eau-dans-huile sont préparées selon le même procédé, tel que décrit ci-dessus, en ne différant des émulsions eau-dans-huile selon l'invention que par la nature de l'agent épaississant, en l'occurrence le Polyélectrolyte anionique (PA₂) différant du Polyélectrolyte anionique (PA₁) par l'absence de monomère associatif dans le squelette polymèriques, l'émulsion correspondante (F'₂) montre une baisse de 34% de la viscosité dynamique deladite émulsion (F'₂) entre le 7^{ème} jour et la fin du 1 er mois de stockage à 20°C. Lorsque la quantité de Polyélectrolyte anionique (PA₂) est augmentée à 2%, contre 0,7% pour le Polyélectrolyte anionique (PA₁), l'émulsion eau-dans-huile obtenue (F'₅) déphase après 3 mois de stockage à 20°C.

Lorsque des émulsions eau-dans-huile sont préparées selon le même procédé tel que décrit ci-dessus, en ne différant des émulsions eau-dans-huile selon l'invention que par la nature de l'agent épaississant, en l'occurrence un polyélectrolyte anionique réticulé à base d'acide acrylique partiellement salifié et d'acide 2-méthyl 2-[(1-oxo 2-propènyl) amino] 1-propanesulfonique partiellement salifié sous forme d'ammonium, lesdites émulsions eau-dans-huile ne présentent pas une consistance liquide ((F'₃) jugée compacte) ou bien se caractérisent par un niveau de viscosité trop élevé (F'₄).

Lorsque une émulsion (F'₆) est préparée selon le même procédé tel que décrit ci-dessus, en ne différant des émulsions eau-dans-huile selon l'invention que par la nature de l'agent épaississant, en l'occurrence un polyélectrolyte de nom INCI Acrylates/C10-30 Alkyl Acrylate Crosspolymer qui est copolymère réticulé de l'acide acrylique et d'un co-monomère hydrophobe, l'émulsion (F'₆) obtenue n'est pas une émulsion eau-dans-huile et elle est instable dès le premier jour de stockage.

### III) Exemples applicatifs

Dans les formules suivantes, les pourcentages sont exprimés en poids de la formulation.

### III-1 Emulsion eau-dans-huile solaire

| | |
|---|---|
| Adipate de di-isopropyle : | 12% |
| Salicylate d'(éthyl hexyle) : | 5% |
| Méthoxycinnamate d'(éthyl hexyle) : | 5% |
| 4-(N,N-diméthyl amino) benzoate d'(éthyl hexyle) : | 8% |
| Butylméthoxydibenzoylméthane : | 2% |
| PEG30 dipolyhydroxystéarate : | 0,4% |
| Fluidanov™ 20X : | 1,2% |
| Polyélectrolyte (PA₁) : | 0,7% |
| Glycérine : | 1,5% |
| Euxyl™ PE9010 : | 1% |
| Eau : | qsp 100% |

### III-2 Emulsion eau-dans-huile soin corps texture coussin

| | |
|---|---|
| Isononanoate d'isononyle : | 4% |
| Parfum : | 0,6% |
| (Ethylhexyl) glycérine : | 0,2% |
| Easynov™ : | 2,5% |
| Polyélectrolyte (PA₁) : | 0,6% |
| Glycérine : | 3% |
| Colorant : | 0,07% |
| Ethanol 95° : | 15% |
| Eau : | qsp 100% |

### III-3 Emulsion eau-dans-huile lait corps grand froid

| | |
|---|---|
| Isononanoate d'isononyle : | 5,3% |
| Parfum : | 0,1% |
| Phénoxyéthanol & (Ethylhexyl) glycérine : | 1% |
| Easynov™ : | 2% |
| Polyélectrolyte (PA₁) : | 1,5% |
| Glycérine : | 14% |
| Eau : | qsp 100% |

### III-4 Emulsion eau-dans-huile soin hydratant

| | |
|---|---|
| Isononanoate d'isononyle : | 6% |
| Fragrance : | 0.1% |
| Phénoxyéthanol & (Ethylhexyl) glycérine : | 1% |
| Easynov™ : | 1.5% |
| Polyélectrolyte (PA₁) : | 0.5% |
| Aquaxyl™ : | 3% |
| Eau : | qsp 100% |

### III-5 Emulsion eau-dans-huile anti-perspirante

| | |
|---|---|
| Isononanoate d'isononyle : | 6% |
| (Ethylhexyl) glycérine : | 1% |
| Easynov™ : | 2,5% |
| Polyélectrolyte (PA₁) : | 0,5% |
| Aquaxyl™ : | 3% |
| Eau : | qsp 100% |
| Chlorhydrate d'aluminium (50%) | 30% |

### III-6 Emulsion eau-dans-huile parfumante

| | |
|---|---|
| Isononanoate d'isononyle : | 6% |
| (Ethylhexyl) glycérine : | 1% |
| Easynov™ : | 2,5% |
| Polyélectrolyte (PA₁) : | 0,5% |
| Aquaxyl™ : | 3% |
| Eau : | qsp 100% |
| Ethanol : | 40% |
| Parfum : | 2,0% |

### III-7 Emulsion eau-dans-huile éclaircissante

| | |
|---|---|
| Isononanoate d'isononyle : | 6% |
| Parfum : | 0,1% |
| Phénoxyéthanol & (Ethylhexyl) glycérine : | 1% |
| Easynov™ : | 1,5% |
| Polyélectrolyte (PA₁) : | 0,5% |
| Sepiwhite™MSH : | 2% |
| Eau : | qsp 100% |

### III-8 Emulsion eau-dans-huile déodorante

| | |
|---|---|
| Isononanoate d'isononyle : | 6% |
| Parfum : | 0,1% |
| Phénoxyéthanol & (Ethylhexyl) glycérine : | 1% |
| Easynov™ : | 1,5% |
| Polyélectrolyte (PA₁) : | 0,5% |
| Lipacide™UG : | 1% |
| Eau : | qsp 100% |

| | |
|---|---|
| Aquaxyl™ (INCl name : Xylitylglucoside & Anhydroxylitol & Xylitol) est un agent hydratant commercialisé par la société SEPPIC. Sepiwhite™MSH (INCl name : ω-undecylenoyl phenylalanine) est un agent éclaircissant de la peau commercialisé par la société SEPPIC. Lipacide™UG (INCl name : undecylenoyl glycine) est un agent déodorant et dermo-purifiant commercialisé par la société SEPPIC. | |

## Revendications

1. Composition (E₁) se présentant sous la forme d'une émulsion eau-dans-huile, **caractérisée en ce qu'**elle comprend pour 100% de sa masse :
- De 60% à 98% massique d'une phase aqueuse (A₁) comprenant pour 100% de sa masse, de 0,05% à 1,65% massique d'un polyélectrolyte anionique réticulé (P) issu de la polymérisation, en présence d'au moins un agent de réticulation, de l'acide 2-méthyl 2-[(1-oxo 2-propènyl) amino] 1-propanesulfonique partiellement ou totalement salifié, avec au moins un monomère neutre choisi parmi les N,N-dialkyl acrylamides, dans lesquels chacun des groupes alkyle comportent entre un et quatre atomes de carbones, et au moins un monomère de formule (I) : 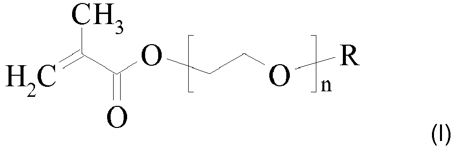 dans laquelle R représente un radical alkyle linéaire ou ramifié comportant de huit à vingt atomes de carbone et n représente un nombre entier supérieur ou égal à un et inférieur ou égal à vingt ;
- De 2% à 40% massique d'une phase grasse (A₂) comprenant pour 100 % de sa masse, de 1,25% à 50% massique d'un système émulsionnant (S), de 50% à 98,75% massique d'au moins une huile et optionnellement d'au moins une cire,
**ladite composition (E₁) étant caractérisée en ce que** ledit système émulsionnant (S) est :
- **Soit** une composition (C₁) d'alkylpolyglycosides représentée par la formule (II) :
R₁-O-(G)ₓ-H (II)
dans laquelle x représente un nombre décimal compris entre 1,05 et 2,5, G représente le reste du xylose, et R₁ représente le radical 2-octyl dodécyle, ladite composition (C₁) consistant en un mélange de composés représentés par les formules (I₁), (I₂), (I₃), (I₄) et (I₅) :
R₁-O-(G)₁-H (II₁)
R₁-O-(G)₂-H (II₂)
R₁-O-(G)₃-H (II₃)
R₁-O-(G)₄-H (II₄)
R₁-O-(G)₅-H (II₅)
dans les proportions molaires respectives a₁, a₂, a₃, a₄ et a₅, telles que :
- La somme a₁+ a₂ + a₃ + a₄ + a₅ est égale à 1 et que
- La somme a₁ + 2a₂ + 3a₃ + 4a₄ + 5a₅ est égale à x,
- **Soit** une composition (C₂) comprenant pour 100% de leur masse :
- De 10 % à 50 % massique d'au moins de la composition (C₁) telle que définie ci-dessus, et
- De 90% à 50% massique d'au moins un alcool gras de formule (III) :
R'₁-OH (III),
dans laquelle R'₁ représente le radical 2-octyl dodécyle ;
- **Soit** une composition (C₃) comprenant pour 100% de sa masse :
De 15 % à 25 % massique d'au moins de la composition (C₁) telle que définie ci-dessus,
- De 55 % à 65 % massique d'au moins un alcool gras de formule (III) :
R'₁-OH (III),
dans laquelle R'₁ représente le radical 2-octyl dodécyle ;
De 10 % à 30 % massique d'au moins un polyhydroxystéarates de polyglycols représenté par la formule (VI) : 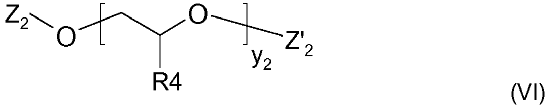 dans laquelle y₂ représente un nombre entier supérieur ou égal à 2 et inférieur ou égal à 50, R₄ représente l'atome d'hydrogène, le radical méthyle ou le radical éthyle, Z₂ représente un radical de formule (VII) : 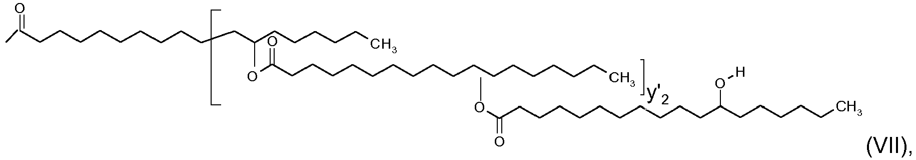 dans laquelle y'₂ représente un nombre entier supérieur ou égal à 0 et inférieur ou égal à 10, plus particulièrement supérieur ou égal à 1 et inférieur ou égal à 10, Z'₂ représente un radical de formule (VII) telle que définie ci-dessus, avec Z₂' identique ou différent de Z₂, ou l'atome d'hydrogène.

2. Composition (E₁) telle que définie à la revendication 1, **caractérisée en ce qu'**elle comprend pour 100% de sa masse :
- De 60% à 98% massique d'une phase aqueuse (A₁) comprenant pour 100% de sa masse, de 0,05% à 1,65% massique d'un polyélectrolyte anionique réticulé (P) issu de la polymérisation, en présence d'un agent de réticulation, de l'acide 2-méthyl 2-[(1-oxo 2-propènyl) amino] 1-propanesulfonique partiellement ou totalement salifié, avec un monomère neutre choisi parmi les N,N-dialkyl acrylamides, dans lesquels chacun des groupes alkyle comportent entre un et quatre atomes de carbones, et un monomère de formule (I) ;
- De 2% à 40% massique d'une phase grasse (A₂) comprenant pour 100 % de sa masse, de 1,25% à 50% massique dudit système émulsionnant (S) ;
- De 50% à 98,75% massique d'une huile et optionnellement d'une cire.

3. Composition (E₁) telle que définie à l'une quelconque des revendications 1 ou 2, **caractérisée en ce que** ledit polyélectrolyte anionique réticulé (P) comporte pour 100% molaire de ses monomères constitutifs :
- De 20% molaire à 80% molaire d'unités monomériques issues dudit acide 2-méthyl 2-[(1-oxo 2-propènyl) amino] 1-propanesulfonique partiellement ou totalement salifiée ;
- De 15% molaire à 75% molaire d'unités monomériques issues d'au moins un monomère neutre choisi parmi les N,N-dialkyl acrylamides, dans lesquels chacun des groupes alkyle comportent entre un et quatre atomes de carbones ;
- De 0,5% à 5% molaire d'unités monomériques issues d'au moins un monomère de formule (I).

4. Composition (E₁) telle que définie à l'une ou quelconque des revendications 1 à 3, **caractérisée en ce que** ledit monomère neutre est le N,N-diméthyl acrylamide.

5. Composition (E₁) telle que définie à l'une ou quelconque des revendications 1 à 4, **caractérisée en ce que** ledit monomère de formule (I) est le méthacrylate de lauryle tétraéthoxylé.

6. Composition (E₁) telle que définie à l'une ou quelconque des revendications 1 à 5, **caractérisée en ce que** ledit polyélectrolyte anionique réticulé (P) est un terpolymère de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate.

7. Composition (E₁) telle que définie à l'une ou quelconque des revendications 1 à 6, **caractérisée en ce que** ledit polyélectrolyte anionique réticulé (P) comporte pour 100% molaire :
- De 60% molaire à 80% molaire d'unités monomériques issues de l'acide 2-méthyl 2-[(1-oxo 2-propènyl) amino] 1-propanesulfonique partiellement salifié sous forme d'ammonium,
- De 15% molaire à 39,5% molaire d'unités monomériques issues du N,N-diméthyl acrylamide, et
- De 0,5% molaire à 5% molaire d'unités monomériques issues du méthacrylate de lauryle tétraéthoxylé.

8. Composition (E₁) telle que définie à l'une quelconque des revendications 1 à 7, **caractérisée en ce qu'**elle comprend en outre, un ou plusieurs composés auxiliaires chois parmi les tensioactifs moussants et/ou détergents, les tensioactifs épaississants et/ou gélifiants, les agents épaississants et/ou gélifiants, les agents stabilisants, les composés filmogènes, les solvants et cosolvants, les agents hydrotropes, les agents plastifiants, les agents émulsionnants et co-émulsionnants, les agents opacificants, les agents nacrants, les agents surgraissants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

9. Composition (E₁) telle que définie à l'une ou quelconque des revendications 1 à 8, **caractérisée en ce que** sa viscosité dynamique, mesurée à une température de 20°C et à l'aide d'un viscosimètre Brookfield de type LVT à une vitesse de 6 tours/minute, est supérieure ou égale à 500mPa.s et inférieure ou égale à 40.000mPa.s.

10. Utilisation de la composition (E₁) telle que définie à l'une des revendications 1 à 8, pour le traitement cosmétique de la peau, des cheveux et/ou des muqueuses.

11. Utilisation de la composition (E₁) telle que définie à la revendication 10, pour le nettoyage, pour la protection et/ou pour le soin de la peau, des cheveux, du cuir chevelu ou des muqueuses.

## Patentansprüche

1. Zusammensetzung (E₁) in Form einer Wasser-in-Öl-Emulsion, **dadurch gekennzeichnet, dass** sie auf 100 % ihrer Masse Folgendes umfasst:
- 60 bis 98 Masse-% einer wässrigen Phase (A₁), welche auf 100 % ihrer Masse 0,05 bis 1,65 Masse-% eines vernetzten anionischen Polyelektrolyts (P) umfasst, der aus der Polymerisation, in Gegenwart von mindestens einem Vernetzungsmittel, von teilweise oder vollständig in ein Salz überführter 2-Methyl-2-[(1-oxo-2-propenyl)-amino]-1-Propansulfonsäure mit mindestens einem neutralen Monomer stammt, ausgewählt aus N,N-Dialkylacrylamiden, in denen jede der Alkylgruppen zwischen ein und vier Kohlenstoffatome umfasst, und mindestens ein Monomer der Formel (I): 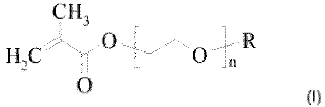 in der R für einen geradkettigen oder verzweigten Alkylrest steht, der acht bis zwanzig Kohlenstoffatome umfasst, und n für eine Ganzzahl größer oder gleich eins und kleiner oder gleich zwanzig steht;
- 2 bis 40 Masse-% einer Fettphase (A₂), welche auf 100 % ihrer Masse 1,25 bis 50 Masse-% eines Emulgatorsystems (S), 50 bis 98,75 Masse-% mindestens eines Öls, und optional mindestens eines Wachses umfasst,
**wobei die Zusammensetzung (E₁) dadurch gekennzeichnet ist, dass** es sich bei dem Emulgatorsystem (S) um Folgendes handelt:
- **entweder** eine Zusammensetzung (C₁) von Alkylpolyglycosiden, für die die Formel (II) steht:
R₁-O-(G)ₓ-H (II)
in der x für eine Dezimalzahl zwischen 1,05 und 2,5 steht, G für den Rest der Xylose steht, und R₁ für den 2-Octyldodecylrest steht, wobei die Zusammensetzung (C₁) aus einer Mischung von Verbindungen besteht, für die die Formeln (I₁), (I₂), (I₃), (I₄), und (I₅) stehen:
R₁-O-(G)₁-H (II₁)
R₁-O-(G)₂-H (II₂)
R₁-O-(G)₃-H (II₃)
R₁-O-(G)₄-H (II₄)
R₁-O-(G)₅-H (II₅)
in den jeweiligen molaren Anteilen a₁, a₂, a₃, a₄ und a₅, sodass:
- die Summe a₁ + a₂ + a₃ + a₄ + a₅ gleich 1 und dass
- die Summe a₁ + 2a₂ + 3a₃ + 4a₄ + 5a₅ gleich x ist,
- **oder** eine Zusammensetzung (C₂), welche auf 100 % ihrer Masse Folgendes umfasst:
- 10 bis 50 Masse-% mindestens der Zusammensetzung (C₁), wie vorstehend definiert, und
- 90 bis 50 Masse-% mindestens eines Fettalkohols der Formel (III):
R'₁-OH (III),
in der R'₁ für den 2-Octyldodecylrest steht;
- **oder** eine Zusammensetzung (C₃), welche auf 100 % ihrer Masse Folgendes umfasst:
- 15 bis 25 Masse-% mindestens der Zusammensetzung (C₁), wie vorstehend definiert,
- 55 bis 65 Masse-% mindestens eines Fettalkohols der Formel (III):
R'₁-OH (III),
in der R'₁ für den 2-Octyldodecylrest steht;
- 10 bis 30 Masse-% mindestens eines Polyglykolpolyhydroxystearats, für das die Formel (VI) steht: 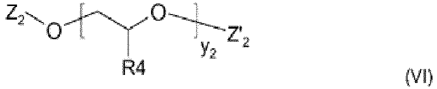 in der y₂ für eine Ganzzahl größer oder gleich 2 und kleiner oder gleich 50 steht, R₄ für das Wasserstoffatom, den Methylrest oder den Ethylrest steht, Z₂ für einen Rest der Formel (VII) steht: 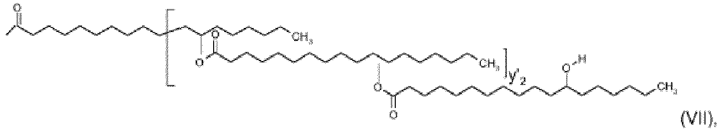 in der y'₂ für eine Ganzzahl größer oder gleich 0 und kleiner oder gleich 10, insbesondere größer oder gleich 1 und kleiner oder gleich 10 steht, Z'₂ für einen Rest der Formel (VII), wie vorstehend definiert, steht, wobei Z₂' gleich oder ungleich Z₂ oder das Wasserstoffatom ist.

2. Zusammensetzung (E₁) nach Anspruch 1, **dadurch gekennzeichnet, dass** sie auf 100 % ihrer Masse Folgendes umfasst:
- 60 bis 98 Masse-% einer wässrigen Phase (A₁), welche auf 100 % ihrer Masse 0,05 bis 1,65 Masse-% eines vernetzten anionischen Polyelektrolyts (P) umfasst, der aus der Polymerisation, in Gegenwart eines Vernetzungsmittels, von teilweise oder vollständig in ein Salz überführter 2-Methyl-2-[(1-oxo-2-propenyl)-amino]-1-Propansulfonsäure mit einem neutralen Monomer stammt, ausgewählt aus N,N-Dialkylacrylamiden, in denen jede der Alkylgruppen zwischen ein und vier Kohlenstoffatome umfasst, und ein Monomer der Formel (I);
- 2 bis 40 Masse-% einer Fettphase (A₂), welche auf 100 % ihrer Masse 1,25 bis 50 Masse-% des Emulgatorsystems (S) umfasst;
- 50 bis 98,75 Masse-% eines Öls und optional eines Wachses.

3. Zusammensetzung (E₁) nach einem der Ansprüche 1 oder 2, **dadurch gekennzeichnet, dass** der vernetzte anionische Polyelektrolyt (P) auf 100 Mol-% seiner aufbauenden Monomere Folgendes umfasst:
- 20 Mol-% bis 80 Mol-% Monomereinheiten, die aus der teilweise oder vollständig in ein Salz überführten 2-Methyl-2-[(1-oxo-2-propenyl)-amino]-1-Propansulfonsäure stammen;
- 15 Mol-% bis 75 Mol-% Monomereinheiten, die aus mindestens einem neutralen Monomer stammen, ausgewählt aus N,N-Dialkylacrylamiden, in denen jede der Alkylgruppen zwischen ein und vier Kohlenstoffatome umfasst;
- 0,5 bis 5 Mol-% Monomereinheiten, die aus mindestens einem Monomer der Formel (I) stammen.

4. Zusammensetzung (E₁) nach einem der Ansprüche 1 bis 3, **dadurch gekennzeichnet, dass** es sich bei dem neutralen Monomer um N,N-Dimethylacrylamid handelt.

5. Zusammensetzung (E₁) nach einem der Ansprüche 1 bis 4, **dadurch gekennzeichnet, dass** es sich bei dem Monomer der Formel (I) um tetraethoxyliertes Laurylmethacrylat handelt.

6. Zusammensetzung (E₁) nach einem der Ansprüche 1 bis 5, **dadurch gekennzeichnet, dass** es sich bei dem vernetzten anionischen Polyelektrolyt (P) um ein teilweise in Form von Ammoniumsalz in ein Salz überführtes Terpolymer der 2-Methyl-2-[(1-oxo-2-propenyl)-amino]-1-Propansulfonsäure, N-N-Dimethylacrylamid und tetraethoxyliertes Laurylmethacrylat handelt, das mit Trimethylolpropantriacrylat vernetzt ist.

7. Zusammensetzung (E₁) nach einem der Ansprüche 1 bis 6, **dadurch gekennzeichnet, dass** der vernetzte anionische Polyelektrolyt (P) auf 100 Mol-% Folgendes umfasst:
- 60 Mol-% bis 80 Mol-% Monomereinheiten, die aus der teilweise in Form von Ammonium in ein Salz überführten 2-Methyl-2-[(1-oxo-2-propenyl)-amino]-1-Propansulfonsäure stammen;
- 15 Mol-% bis 39,5 Mol-% Monomereinheiten, die aus dem N,N-Dimethylacrylamid stammen, und
- 0,5 Mol-% bis 5 Mol-% Monomereinheiten, die aus dem tetraethoxylierten Laurylmethacrylat stammen.

8. Zusammensetzung (E₁) nach einem der Ansprüche 1 bis 7, **dadurch gekennzeichnet, dass** sie des Weiteren eine oder mehrere Hilfsverbindungen umfasst, ausgewählt aus schaumbildenden und/oder reinigenden Tensiden, verdickenden und/oder gelbildenden Tensiden, Verdickungs- und/oder Geliermitteln, Stabilisatoren, filmbildenden Verbindungen, Lösungs- und Co-Lösungsmitteln, Hydrotropen, Weichmachern, Emulgatoren und Co-Emulgatoren, Trübungsmitteln, Perlglanzmitteln, Überfettungsmitteln, Maskierungsmitteln, Chelatbildnern, Antioxidationsmitteln, Duftstoffen, ätherischen Ölen, Konservierungsmitteln, Konditionierungsmitteln, Desodorierungsmitteln, Bleichmitteln, die zum Aufhellen der Haare und der Haut bestimmt sind, Wirkstoffen, die dazu bestimmt sind, eine behandelnde und/oder schützende Wirkung gegenüber der Haut oder den Haaren herbeizuführen, Sonnenschutzfiltern, mineralischen Füllstoffen oder Pigmenten, Partikeln, die einen optischen Effekt verschaffen oder zur Verkapselung von Wirkstoffen bestimmt sind, exfoliativen Partikeln, Texturiermitteln, optischen Aufhellern, Insektenabwehrmitteln.

9. Zusammensetzung (E₁) nach einem der Ansprüche 1 bis 8, **dadurch gekennzeichnet, dass** ihre dynamische Viskosität, gemessen bei einer Temperatur von 20 °C und mithilfe eines Brookfield-Viskosimeters des Typs LVT bei einer Geschwindigkeit von 6 Umdrehungen/Minute, größer oder gleich 500 mPA.s und kleiner oder gleich 40.000 mPA.s ist.

10. Verwendung der Zusammensetzung (E₁) nach einem der Ansprüche 1 bis 8 zur kosmetischen Behandlung der Haut, der Haare und/oder der Schleimhäute.

11. Verwendung der Zusammensetzung (E₁) nach Anspruch 10 zur Reinigung, zum Schutz und/oder zur Pflege der Haut, der Haare, der Kopfhaut oder der Schleimhäute.

## Claims

1. Composition (E₁) in the form of a water-in-oil emulsion, **characterised in that** it comprises, for 100% of its mass:
60% to 98% w/w aqueous phase (A₁), comprising, for 100% of its mass, 0.05% to 1.65% w/w crosslinked anionic polyelectrolyte (P) issuing from the polymerisation, in the presence of at least one crosslinking agent, of 2-methyl 2-[(1-oxo 2-propenyl) amino] 1-propanesulfonic acid, partially or totally salified, with at least one neutral monomer chosen from N,N-dialkyl acrylamides, in which each of the alkyl groups comprise between one and four carbon atoms, and at least one monomer of formula (I) 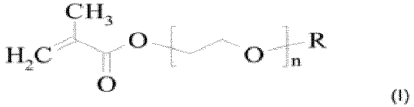 in which R represents a linear or branched alkyl radical comprising from eight to twenty carbon atoms and n represents an integer number greater than or equal to one and less than or equal to twenty;
- 2% to 40% w/w fatty phase (A₂) comprising, for 100% of its mass, 1.25% to 50% w/w emulsifying system (S), 50% to 98.75% w/w at least one oil and optionally at least one wax,
said composition (E₁) being **characterised in that** said emulsifying system (S) is:
- either an alkylpolyglycoside composition (C₁) represented by formula (II):
R₁-O-(G)ₓ-H (II)
in which x represents a decimal number between 1.05 and 2.5, G represents the rest of the xylose, and R₁ represents the 2-octyl dodecyl radical, said composition (C₁) consisting of a mixture of compounds represented by the formulae (I₁), (I₂), (I₃), (I₄) and (I₅):
R₁-O-(G)₁-H (II₁)
R₁-O-(G)₂-H (II₂)
R₁-O-(G)₃-H (II₃)
R₁-O-(G)₄-H (II₄)
R₁-O-(G)₅-H (II₅)
in the respective molar proportions a₁, a₂, a₃, a₄ and a₅, such that:
- the sum a₁ + a₂ + a₃ + a₄ + a₅ is equal to 1, and
- the sum a₁ + 2a₂ + 3a₃ + 4a₄ + 5a₅ is equal to x,
- or a composition (C₂), for 100% of their mass:
- 10% to 50% w/w at least the composition (C₁) as defined above, and
- 90% to 50% w/w at least one fatty alcohol of formula (III):
R'₁-OH (III)
in which R'₁ represents the 2-octyl dodecyl radical;
- or a composition (C₃) comprising, for 100% of its mass:
- 15% to 25% w/w at least the composition (C₁) as defined above,
- 55% to 65% w/at least one fatty alcohol of formula (III):
R'₁-OH (III)
in which R'₁ represents the 2-octyl dodecyl radical;
- 10% to 30% w/w at least one polyglycol polyhydroxy stearate represented by formula (VI) 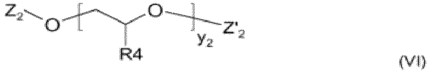 in which y₂ represents an integer number greater than or equal to 2 and less than or equal to 50, R₄ represents the hydrogen atom, the methyl radical or the ethyl radical, Z₂ represents a radical of formula (VII): 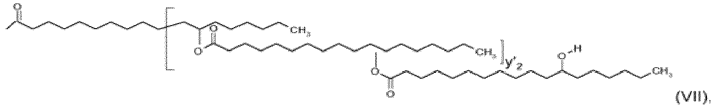 in which y'₂ represents an integer number greater than or equal to 0 and less than or equal to 10, more particularly greater than or equal 1 and less than or equal to 10, Z'₂ represents a radical of formula (VII) as defined above, with Z₂' identical to or different from Z₂, or the hydrogen atom.

2. Composition (E₁) as defined in claim 1, **characterised in that** it comprises, for 100% of its mass:
- 60% to 98% w/w an aqueous phase (A₁) comprising, for 100% of its mass, 0.05% to 1.65% w/w crosslinked ionic polyelectrolyte (P) issuing from the polymerisation, in the presence of a crosslinking agent, of 2-methyl 2-[(1-oxo 2-propenyl) amino] 1-propanesulfonic acid, partially or totally salified, with a neutral monomer chosen from N,N-dialkyl acrylamides, in which each of the alkyl groups comprise between one and four carbon atoms, and a monomer of formula (I);
- 2% to 40% w/w a fatty phase (A₂) comprising, for 100% of its mass, 1.25% to 50% w/w of said emulsifying system (S);
- 50% to 98.75% w/w oil and optionally a wax.

3. Composition (E₁) as defined in either claim 1 or claim 2, **characterised in that** said crosslinked anionic polyelectrolyte (P) comprises, for 100% molar of its constituent monomers:
- 20% molar to 80% molar monomeric units issuing from said partially or totally salified 2-methyl 2-[(1-oxo 2-propenyl) amino] 1-propanesulfonic acid;
- 15% molar to 75% molar monomeric units issuing from at least one neutral monomer chosen from N,N-dialkyl acrylamides, in which each of said alkyl groups comprise between one and four carbon atoms;
- 0.5% to 5% molar monomeric units issuing from at least one monomer of formula (I).

4. Composition (E₁) as defined in one or any of claims 1 to 3, **characterised in that** said neutral monomer is N,N-dimethyl acrylamide.

5. Composition (E₁) as defined in one or any of claims 1 to 4, **characterised in that** said monomer of formula (I) is tetraethoxylated lauryl methacrylate.

6. Composition (E₁) as defined in one or any of claims 1 to 5, **characterised in that** said crosslinked anionic polyeletrolyte (P) is a terpolymer of 2-methyl 2-[(1-oxo 2-propenyl) amino] 1-propanesulfonic acid partially salified in the form of ammonium salt, N,N-dimethyl acrylamide and tetraethoxylated lauryl methacrylate, crosslinked with trimethylolpropane triacrylate.

7. Composition (E₁) as defined in one or any of claims 1 to 6, **characterised in that** said crosslinked anionic polyelectrolyte (P) comprises, for 100% molar:
- 60% molar to 80% molar monomeric units issuing from 2-methyl 2-[(1-oxo 2-propenyl) amino] 1-propanesulfonic acid partially salified in ammonium form,
- 15% molar to 39.5% molar monomeric units issuing from N,N-dimethyl acrylamide, and
- 0.5% molar to 5% molar monomeric units issuing from tetraethoxylated lauryl methacrylate.

8. Composition (E₁) as defined in any of claims 1 to 7, **characterised in that** it further comprises one or more auxiliary compounds chosen from foaming and/or detergent surfactants, thickening and/or gelling surfactants, thickening and/or gelling agents, stabilisers, film-forming compounds, solvents and co-solvents, hydrotropes, plasticisers, emulsifiers and co-emulsifiers, opacifiers, pearling agents, superfatting agents, sequestering agents, chelating agents, antioxidants, perfumes, essential oils, preservatives, conditioners, deodorisers, bleaches intended for bleaching hair and skin, active principles intended to provide a treating and/or protective action vis-à-vis the skin or hair, sun filters, mineral fillers or pigments, particles procuring a visual effect or intended for the encapsulation of active agents, exfoliant particles, texture agents, optical brighteners, and insect repellents.

9. Composition (E₁) as defined in one or any of claims 1 to 8, **characterised in that** its dynamic viscosity, measured at a temperature of 20°C and by means of a Brookfield viscometer of the LVT type at a speed of 6 revolutions/minute, is greater than or equal to 500 mPa.s and less than or equal to 40,000 mPa.s.

10. Use of the composition (E₁) as defined in one of claims 1 to 8, for the cosmetic treatment of the skin, hair and/or mucous membranes.

11. Use of the composition (E₁) as defined in claim 10, for the cleaning, protection and/or care of the skin, hair, scalp or mucous membranes.
